# EUROPEAN PATENT SPECIFICATION

(11) **EP 4 072 476 B1**
(45) Date of publication and mention of the grant of the patent: **21.05.2025**
(21) Application number: 20828770.6
(22) Date of filing: 10.12.2020
(51) Int. Cl.: A61F 2/44, A61F 2/46

(54) **INTERSOMATIC CAGE FOR VERTEBRAL STABILIZATION**
INTERSOMATISCHER KÄFIG ZUR WIRBELSTABILISIERUNG
CAGE INTERSOMATIQUE POUR STABILISATION VERTÉBRALE

(30) Priority: 13.12.2019 IT 201900023913
(43) Date of publication of application: 19.10.2022
(73) Proprietor: SPS S.r.l., 10010 Scarmagno (TO) (IT)
(72) Inventor: LAURENTI, Riccardo, 10010 Scarmagno (Torino) (IT)
(74) Representative: Buzzi, Notaro & Antonielli d'Oulx S.p.A.
(86) International application number: PCT/IB2020/061749
(87) International publication number: WO 2021/116972

(56) References cited:
- WO-A1-2009/029929
- WO-A1-2009/144562
- WO-A1-2016/207798
- WO-A2-2018/112324
- DE-U1- 202011 000 202
- US-A1- 2003 004 576
- US-A1- 2013 006 361

## Description

### Field of the invention

The present invention relates to an intersomatic cage for vertebral stabilization designed to be inserted between two contiguous vertebrae so as to space them apart and thus keep them at a mutual distance such to restore the intervertebral space, creating the decompression of the nerve roots and the acceleration of the intersomatic fusion.

### State of the prior art

Intersomatic cages thus made typically comprise a body provided with external anchoring formations and with a nose protruding from the front end of the body and designed to be inserted between two contiguous vertebrae during the surgical insertion of the intersomatic cage.

In the case of document EP1408891, the body of the cage is cylindrical-shaped and the nose has an elliptical cross-section with variable thickness, decreasing toward the free end thereof.

In the case of documents WO2009/144562 and EP2231074, the body is generally parallelepiped-shaped with two respectively upper and lower opposite faces, with reference to the implanted position of the cage in the intervertebral space of a subject with an upright spine, provided with the aforementioned anchoring formations, and the nose is arranged according to an angle relative to such opposite faces. In the case of WO2009/144562 the nose is inclined from 40° to 60° with respect to the horizontal plane of the cage parallel to the upper and lower faces thereof and the cross-section thereof has variable thickness, decreasing toward the opposite sides of the body of the cage. In the case of EP2231074, the nose is arranged, orthogonally to the upper and lower faces of the body of the cage, in offset position, i.e. it is laterally staggered with respect to the central zone of the front end of the body.

Similar intersomatic cages are also described and illustrated in documents WO2016/207798A1 and WO2009/029929A1.

All these solutions have a common drawback relating to the difficulty during the surgical insertion of the cage, when performing the tasks required to introduce the nose into the intervertebral space and thus the subsequent wedging of the body of the cage so as to position the upper and lower faces of the body, with the relative anchoring formations, between the two vertebrae.

### Summary of the invention

The object of the present invention is to solve the aforementioned problem and in particular to make the procedure for inserting the intersomatic cage simpler, easier and safer. The present invention relates to an intersomatic cage as claimed hereafter. Preferred embodiments of the invention are set forth in the dependent claims.

According to the invention, this object is achieved essentially due to the fact that the nose extends parallel to the two upper and lower opposite faces of the body of the cage and it has a substantially quadrangular shape with a constant cross-section and rounded edges.

These distinctive characteristics relating to the position and shape of the nose of the cage allow to considerably simplify the surgical insertion of the cage. Specifically, the position of the nose - which according to the invention extends from a median area of the front end of the body - allows to perform vertebral distraction, after inserting the nose into the intervertebral space, by rotating the cage by about 90° alternately in a clockwise and anti-clockwise direction, without having to worry about what the correct direction of rotation should be: thus, the task is much simpler and more intuitive. The shape of the nose, quadrangular with constant cross-section over the entire length thereof, allows to perform the vertebral distraction with greater safety, avoiding the risk of slipping when introducing the nose into the intervertebral space, as opposed to what could occur if the nose had a variable cross-section with decreasing thickness toward the free end thereof.

Further and advantageous secondary characteristics of the intersomatic cage according to the invention lie in the fact that the nose is connected to the median area of the front end of the body through curved surfaces having variable radiuses, and in the fact that the body has a slightly decreasing thickness from the front end thereof toward the rear end. Such rear end of the body is formed with a recess for engaging a surgical instrument for the insertion of the cage, by means of which the operator can easily perform the aforementioned clockwise and anti-clockwise rotation tasks.

### Brief description of the drawings

The invention will now be described in detail with reference to the attached drawings, provided purely by way of non-limiting example, wherein:

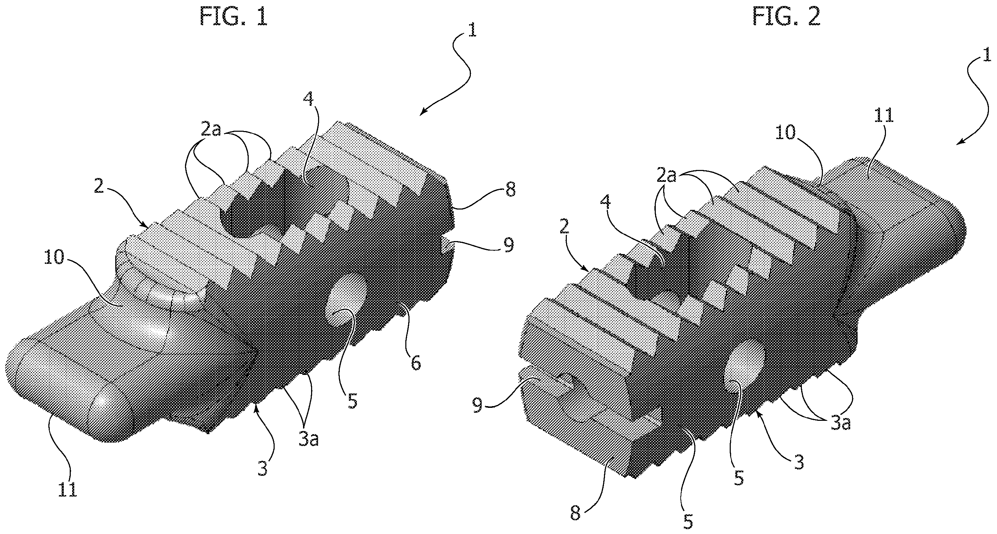

- figure 1 is a front perspective view of the intersomatic cage according to the invention,
- figure 2 is a dorsal perspective view of the cage,
- figure 3 is a top plan view of the cage,
- figure 4 is a side elevational view of the cage,

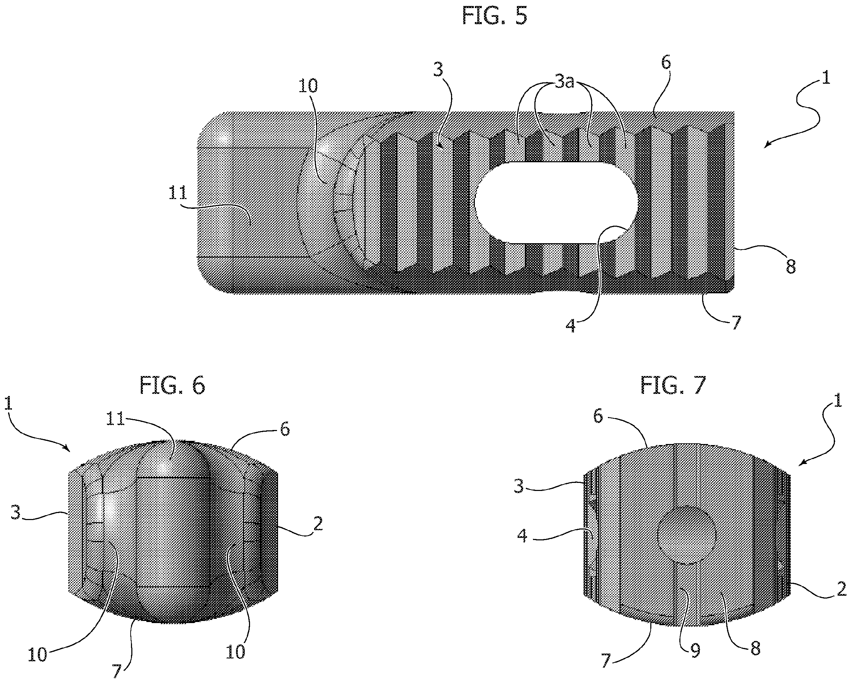

- figure 5 is a bottom plan view of the cage,
- figure 6 is a front elevational view of figure 5, and
- figure 7 is a dorsal view of figure 5.

### Detailed description of the invention

With reference to the figures, the intersomatic cage according to the invention consists of a generally prismatic-shaped and more precisely substantially parallelepiped-shaped monolithic body 1, having an upper face 2 and a lower face 3 (with reference to the implanted position of the cage in the intervertebral space of a subject with an upright spine), both formed with respective anchoring formations consisting of parallel indentations 2a, 3a. The faces 2 and 3 are not precisely parallel but are slightly inclined, and for this reason the parallelepiped shape of the body 1 is approximate.

A slot-shaped through opening 4 vertically traverses the body 1 between the upper 2 and lower 3 faces, and a circular through hole 5 extends between the lateral walls 6, 7 of the body 1.

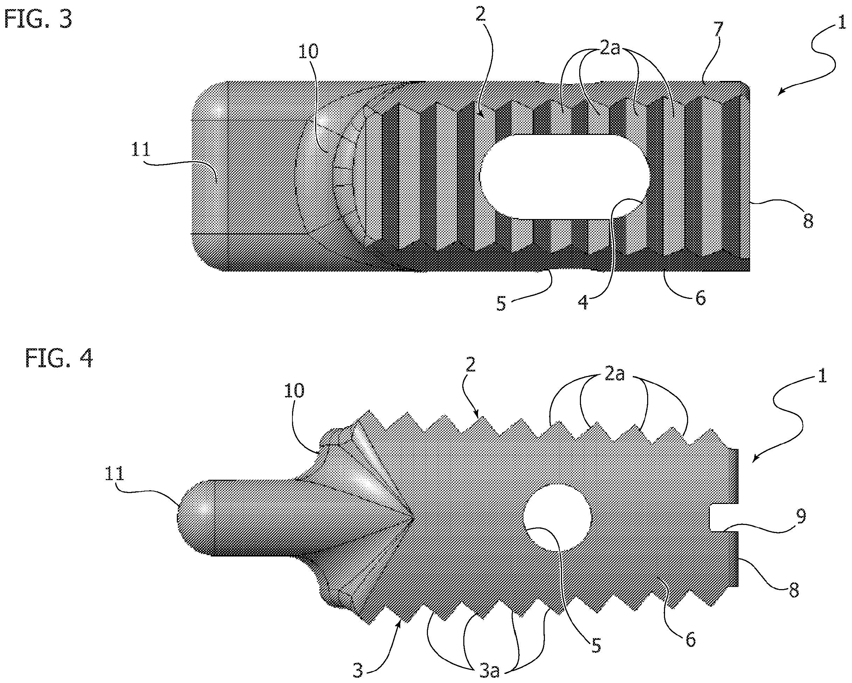

As observable in figure 4, the body 1 has a height, measured between the upper 2 and lower 3 faces, which decreases slightly toward the rear end thereof, indicated with 8 and in which a recess 9 for the introduction of an instrument for the surgical insertion of the cage into the intervertebral space. This instrument, not illustrated, is configured so as to engage axially and torsionally with the recess 9 so as to be able to rotate the body 1 alternately in a clockwise and anti-clockwise direction while being simultaneously pushed.

A median longitudinal plane between the upper and lower faces thereof can be identified in the body 1 (figure 4).

A nose 11 - which has the function of paving the way by dilating the intervertebral space during the process for inserting the intersomatic cage - protrudes from the front end of the body 1, indicated with reference numeral 10. According to the invention, the nose 11 extends parallel to the upper 2 and lower 3 faces of the body 1 and it has a substantially quadrangular shape with a constant cross-section and rounded edges. More precisely, the nose 11 extends on the extension of the aforementioned median longitudinal plane of the body 1 and thus protrudes from a median area of the front end 10, connecting to such median area through curved surfaces having variable radiuses, clearly visible in the figures.

Thanks to the shape and position described above, during the surgical insertion of the cage the nose 11 allows to safely perform the vertebral distraction, avoiding any risk of the body 1 slipping. The vertebral distraction can thus be performed more easily and safely by rotating the cage by about 90° alternately in one direction and in the opposite direction, hence considerably simplifying the insertion procedure since the operator can achieve the object without having to worry about the direction of rotation, unlike the case of intersomatic cages according to the aforementioned prior art.

Obviously, the construction details and the embodiments may widely vary with respect to what has been described and illustrated, without departing from the scope of protection of the invention as described in the claims that follow.

## Claims

1. Intersomatic cage for vertebral stabilization, comprising a generally parallelepiped monolithic body (1) having two respectively upper and lower opposite faces (2, 3), with reference to the implanted position of the cage in an intervertebral space of a subject with an upright spine, provided with anchoring formations (2a, 3a), said body (1) having a median longitudinal plane between said upper and lower faces (2, 3) and a nose (11) protruding from the front end (10) of the body (1) and designed to be inserted between two contiguous vertebrae so as to space them apart during the surgical insertion of the cage, wherein:
- the nose (11) extends from a median area of said front end (10) of the body (1) on the extension of said median longitudinal plane of the body (1) parallel to said upper and lower faces (2, 3),
- the nose (11) cross section in a plane perpendicular to said median longitudinal plane is substantially constant and has a quadrangular shape with rounded edges,
- the nose (11) is connected to said median area of said front end (10) of the body (1) through curved surfaces having variable radiuses.

2. Cage according to claim 1, **characterised in that** the body (1) has a slightly decreasing thickness from said front end (10) toward the rear end (8).

3. Cage according to one or more of the preceding claims, **characterised in that** the body (1) has openings (4, 5) at said upper and lower faces (2, 3) as well as at the lateral faces (6, 7) thereof.

4. Cage according to one or more of the preceding claims, **characterised in that** said anchoring formations consist of parallel indentations (2a, 3a).

5. Cage according to one or more of the preceding claims, **characterised in that** the rear end (8) of the body (1) is formed with a recess (9) for engaging a surgical instrument for the insertion of the cage.

## Patentansprüche

1. Intersomatischer Käfig für Wirbelstabilisierung, umfassend einen allgemein quaderförmigen monolithischen Körper (1) mit zwei oberen bzw. unteren gegenüberliegenden Flächen (2, 3), mit Bezug auf die implantierte Position des Käfigs in einem Zwischenwirbelraum eines Individuums mit einer aufrechten Wirbelsäule, die Verankerungsformationen (2a, 3a) aufweisen, wobei der Körper (1) eine mediane Längsebene zwischen den oberen und unteren Flächen (2, 3) und eine Nase (11), die vom vorderen Ende (10) des Körpers (1) vorragt und zur Einführung zwischen zwei aneinandergrenzenden Wirbeln ausgelegt ist, um diese während der chirurgischen Einführung des Käfigs auseinanderzuhalten, aufweist,
wobei:
- sich die Nase (11) von einem medianen Bereich des vorderen Endes (10) des Körpers (1) auf der Verlängerung der medianen Längsebene des Körpers (1) parallel zu den oberen und unteren Flächen (2, 3) erstreckt,
- der Querschnitt der Nase (11) in einer Ebene senkrecht zur medianen Längsebene im Wesentlichen konstant ist und eine viereckige Form mit abgerundeten Ecken aufweist,
- die Nase (11) durch gebogene Oberflächen, die variable Radien aufweisen, mit dem medianen Bereich des vorderen Endes (10) des Körpers (1) verbunden ist.

2. Käfig nach Anspruch 1, **dadurch gekennzeichnet, dass** der Körper (1) eine leicht abnehmende Dicke vom vorderen Ende (10) zum hinteren Ende (8) aufweist.

3. Käfig nach ein oder mehr der vorhergehenden Ansprüche, **dadurch gekennzeichnet, dass** der Körper (1) Öffnungen (4, 5) an den oberen und unteren Flächen (2, 3) sowie an den Seitenflächen (6, 7) davon aufweist.

4. Käfig nach ein oder mehr der vorhergehenden Ansprüche, **dadurch gekennzeichnet, dass** die Verankerungsformationen aus parallelen Vertiefungen (2a, 3a) bestehen.

5. Käfig nach ein oder mehr der vorhergehenden Ansprüche, **dadurch gekennzeichnet, dass** das hintere Ende (8) des Körpers (1) mit einer Aussparung (9) für einen Eingriff mit einem chirurgischen Instrument zur Einführung des Käfigs ausgebildet ist.

## Revendications

1. Cage intersomatique pour stabilisation vertébrale, comprenant un corps monolithique (1) généralement parallélépipédique ayant deux faces opposées respectivement supérieure et inférieure (2, 3), en référence à la position implantée de la cage dans un espace intervertébral d'un sujet à colonne vertébrale droite, pourvu de formations d'ancrage (2a, 3a), ledit corps (1) présentant un plan longitudinal médian entre lesdites faces supérieure et inférieure (2, 3) et un nez (11) faisant saillie de l'extrémité avant (10) du corps (1) et conçu pour être inséré entre deux vertèbres contiguës de manière à les écarter lors de l'insertion chirurgicale de la cage,
dans laquelle :
- le nez (11) s'étendant à partir d'une zone médiane de ladite extrémité avant (10) du corps (1) sur le prolongement dudit plan longitudinal médian du corps (1) parallèle auxdites faces supérieure et inférieure (2, 3),
- la section transversale du nez (11) dans un plan perpendiculaire audit plan longitudinal médian étant sensiblement constante et ayant une forme quadrangulaire à bords arrondis,
- le nez (11) étant relié à ladite zone médiane de ladite extrémité avant (10) du corps (1) par des surfaces courbes à rayons variables.

2. Cage selon la revendication 1, **caractérisée en ce que** le corps (1) présente une épaisseur légèrement décroissante de ladite extrémité avant (10) vers l'extrémité arrière (8).

3. Cage selon une ou plusieurs des revendications précédentes, **caractérisée en ce que** le corps (1) présente des ouvertures (4, 5) au niveau desdites faces supérieure et inférieure (2, 3) ainsi qu'au niveau de leurs faces latérales (6, 7).

4. Cage selon une ou plusieurs des revendications précédentes, **caractérisée en ce que** lesdites formations d'ancrage consistent en des échancrures parallèles (2a, 3a).

5. Cage selon une ou plusieurs des revendications précédentes, **caractérisée en ce que** l'extrémité arrière (8) du corps (1) est formée d'un renfoncement (9) pour engager un instrument chirurgical pour l'insertion de la cage.
